# EUROPÄISCHE PATENTSCHRIFT

(11) **EP 2 105 112 B1**
(45) Veröffentlichungstag und Bekanntmachung des Hinweises auf die Patenterteilung: **05.01.2011**
(21) Anmeldenummer: 09003539.5
(22) Anmeldetag: 11.03.2009
(51) Int. Cl.: A61F 5/01

(54) **Orthopädische Vorrichtung zur Korrektur von Zehenfehlstellungen**
Orthopaedic device for correcting defective toe positions
Dispositif orthopédique pour corriger des malpositions des orteils

(30) Priorität: 27.03.2008 DE 202008004214 U
(43) Veröffentlichungstag der Anmeldung: 30.09.2009
(73) Patentinhaber: Hallufix AG, 81925 München (DE)
(72) Erfinder: Krauss, Axel, 81541 München (DE)
(74) Vertreter: Thoma, Michael

(56) Entgegenhaltungen:
- EP-A- 1 531 768
- EP-A- 1 568 337

## Beschreibung

Die vorliegende Erfindung betrifft eine orthopädische Vorrichtung zur Korrektur von Zehenfehlstellungen, insbesondere von Hallux valgus, mit einer Gelenkschiene, die zwei um eine liegende Achse miteinander gelenkig verbundene Schienenschenkel aufweist, von denen ein hinterer Schienenschenkel mittels einer Mittelfußbandage am Mittelfuß befestigbar und von denen ein vorderer Schienenschenkel mittels einer Zehenbefestigung mit einer Zehe verbindbar ist.

Durch verschiedene Ursachen, insbesondere das Tragen zu enger und zu hoher Schuhe kann es zu schmerzhaften Zehenfehlstellungen kommen, die oftmals operativ behandelt werden. Oftmals ist es dabei insbesondere die Großzehe, die eine zur Fußaußenseite hin gebogene Fehlstellung einnimmt, die einhergeht mit Gewebeveränderungen im Bereich des Großzehengrundgelenks bzw. des Mittelfußes.

Zur Korrektur derartiger Zehenfehlstellungen, insbesondere Hallux valgus, wurde bereits vorgeschlagen, am Fuß eine Schiene anzubringen, mittels derer die zu korrigierende Zehe sozusagen zurechtgebogen wird, d.h. in ihre Sollstellung bzw. zu ihrer Sollstellung hin gezwungen bzw. dort gehalten wird. Dabei werden entsprechende Schienen einerseits im postoperativen Bereich eingesetzt, um die Fußglieder nach einem operativen Korrektureingriff zu stabilisieren und in der Sollstellung zu halten. Zum anderen jedoch wurde auch vorgeschlagen, Zehenfehlstellungen der genannten Art ohne operativen Eingriff konservativ zu therapieren. Hierzu werden Korrekturschienen verwendet, die einerseits nachts getragen werden können, andererseits jedoch auch tagsüber beim Laufen und insbesondere auch in Schuhen getragen werden können.

Um hier die Abrollbewegung des Fußes nicht zu sehr zu behindern, wurden Gelenkschienen vorgeschlagen, die zwei durch eine liegende Gelenkachse verbundene Schienenschenkel umfassen. Die Gelenksachse erstreckt sich hierbei im Wesentlichen koaxial zur Zehengelenksgrundachse, so dass der vorderere Schienenschenkel mit den Zehen gegenüber dem hinteren Schienenschenkel abgewinkel werden kann, ohne dass es zu einer großen Relativbewegung zwischen Schiene und Fußgliedern kommt. Eine derartige Schiene zeigt beispielsweise das europäische Patent EP 1531768. Die Gelenkschiene erstreckt sich dabei auf der Fußinnenseite, wobei das Gelenk zwischen den beiden Schienenschenkel schalenförmig konturiert und an die ballenförmige Wölbung des Großzehengrundgelenks angepasst ist, auf dem die genannte Gelenksschale sitzt, um auch dort das Gewebe zu unterstützen. Die Großzehe wird mittels einer Zehenbandage zur Fußinnenseite hin gezogen, wodurch die Gelenkschiene auch im Bereich des Großzehengrundgelenks infolge der Reaktionskräfte auf das Gewebe gedrückt wird. Von Vorteil ist bei dieser bekannten Gelenksschienenausbildung die Tatsache, dass die Fußsohle freibleibt, d.h. keine sohlenähnlichen Trittstücke auf der Fußsohlenseite das Laufen erschweren. Der Patient kann auch mit angelegter Gelenksschiene wie barfuß laufen bzw. ohne Behinderung in einen Schuh steigen.

Der vorliegenden Erfindung liegt die Aufgabe zugrunde, eine verbesserte orthopädische Vorrichtung der eingangs genannten Art zu schaffen, die Nachteile des Standes der Technik vermeidet und Letzteren in vorteilhafter Weise weiterbildet. Insbesondere soll eine effiziente und stabile Zehenkorrektur erreicht werden, ohne hierbei unerwünscht starken Druck auf das Gewebe im Bereich des Großzehengrundgelenks auszüben, wobei gleichzeitig ein angenehmes und leichtes Tragen sowohl nachts als auch tagsüber in Schuhen ohne Behinderung beim Laufen sichergestellt sein soll.

Erfindungsgemäß wird diese Aufgabe durch eine orthopädische Vorrichtung gemäß Anspruch 1 gelöst. Bevorzugte Ausgestaltungen sind Gegenstand der abhängigen Ansprüche.

Es wird also vorgeschlagen, von der Fußaußenseite her zu arbeiten. Entgegen den bislang verfolgten Ansätzen wird nicht die Fußinnenseite, sondern die Fußaußenseite geschient und eine jeweils zu korrigierende Zehe von der auf der Fußaußenseite positionierten Gelenkschiene her in eine Sollstellung gezwungen bzw. dort gehalten. Erfindungsgemäß erstrecken sich beide Schienenschenkel auf der Fußaußenseite, wobei der vordere Schienenschenkel eine Querstrebe trägt, die sich quer zu der Gelenkschiene über und/oder unter zumindest dem kleinen Zehen hinweg zur Fußinnenseite hin zu einer zu korrigierenden Zehe erstreckt, die mittels der genannten Zehenbefestigung an der Querstrebe befestigbar ist. Durch die auf der Fußaußenseite platzierte Gelenkschiene bleibt die Fußinnenseite und das Gewebe am Fußzehengrundgelenk im Wesentlichen druckfrei, wobei durch das Freibleiben der Fußinnenseite der Tragekomfort in Schuhen wesentlich erhöht werden kann, da üblicherweise gerade dort durch die Abrollbewegung des Fußes stärkere Reibbewegungen zum Schuhkorpus auftreten.

Insbesondere kann die genannte Querstrebe als Druckstrebe ausgebildet sein, die über geeignete Eingriffsmittel mit der zu korrigierenden Zehe in Eingriff steht, um diese von der Fußaußenseite her lateral nach innen zu drücken, d.h. die genannte Druckstrebe drückt die zu korrigierende Zehe zur Fußinnenseite hin. Eine derartige Korrekturkraft ist insbesondere bei Hallux valgus-Fehlstellungen der Großzehe hilfreich, um die Großzehe zurück in ihre gerade Sollstellung zu drücken. Je nach Fehlstellung der zu korrigierenden Zehe kann die genannte Querstrebe jedoch auch als Zug- und Druckstrebe ausgebildet sein, um auf eine jeweils zu korrigierende Zehe Zug- und/oder Druckkräfte auszüben. Beispielsweise können die kleine Zehe oder die hierzu benachbarte Zehe, die oftmals durch zu schmale Schuhe eine Fehlstellung zur Fußinnenseite hin aufweisen, durch die auf der Fußaußenseite platzierte Gelenkschiene und die daran befestigte Querstrebe zurück nach außen gezogen werden, während gleichzeitig oder alternativ die Großzehe zur Fußinnenseite hin gedrückt werden kann.

Um von der genannten Querstrebe aus zur Fußinnenseite hin gerichtete Korrekturkräfte auf eine jeweilige Zehe ausüben zu können, können an der Druckstrebe verschiedene Eingriffsmittel vorgesehen sein. Beispielsweise könnte sich die Druckstrebe über die zu korrigierende Zehe hinaus zur Fußinnenseite erstrecken und eine Zugschlinge beispielsweise in Form einer Bandage aufweisen, die die Zehe zur Fußinnenseite hin zieht. In vorteilhafter Weiterbildung der Erfindung jedoch kann die genannte Querstrebe einen Eingriffssteg aufweisen, der sich zwischen einem Paar benachbarter Zehen im Wesentlichen vertikal erstreckt und in den Zwischenraum zwischen die Zehen hineinspringt. Dieser Steg kann die auf der Fußinnenseite befindliche Zehe zur Fußinnenseite hin drücken und/oder die auf der Fußaußenseite befindliche Zehe mittels einer geeigneten Zugbandage zur Fußinnenseite hin ziehen. Der genannte Positioniersteg, der sich zwischen zwei benachbarten Zehen erstreckt, bildet insofern Druckmittel zum Nach-innen-drücken einer Zehe. Ggf. können an dem Drucksteg mehrere solcher Positionierstege vorhanden sein, um mehrere Zehen zur Fußinnenseite hin zu drücken.

Um einen besonders angenehmen Tragekomfort zu erreichen, ist dabei in vorteilhafter Weiterbildung der Erfindung vorgesehen, dass die genannte Druckstebe von der Fußaußenseite her betrachtet vor der Großzehe endet, so dass sich keine starre Strebe unter bzw. über der Großzehe erstreckt. Das Aussparen der Großzehe bewirkt überraschenderweise eine besonders große Erhöhung des Tragekomforts, da beim Laufen das zur Sohle hin Freibleiben der Großzehe besonders angenehm empfunden wird. Nichtsdestotrotz kann die Großzehe bei Hallux valgus-Fehlstellungen stabil und effizient korrigiert werden, indem die Druckstrebe einen seitlich vor der Großzehe in den Zehenzwischenraum hinein vorspringenden Positioniersteg aufweist, mittels dessen die Großzehe zur Fußinnenseite hin gedrückt werden kann.

In Weiterbildung der Erfindung wird die zu korrigierende Zehe mittels einer Zehenbandage an der Druckstrebe befestigt, wobei die genannte Zehenbandage vorteilhafterweise an dem in den Zehenzwischenraum hinein vorspringenden Positioniersteg angelenkt sein kann, insbesondere an diesem durchgeschlauft sein kann.

Der genannte Positioniersteg kann grundsätzlich starr an der Druckstrebe befestigt sein. Eine Abrollbewegung der daran befestigten Zehe ist trotzdem gewährleistet, da die gesamte Querstrebe zusammen mit dem vorderen Schienenschenkel um die Gelenkachse zwischen den beiden Schienenschenkel abgeknickt werden kann. Zusätzlich kann jedoch auch der genannte Positioniersteg gegenüber der Querstrebe nochmals gelenkig gelagert sein, insbesondere durch eine Gelenkachse, die sich im Wesentlichen parallel zu der von der Querstrebe definierten Querachse erstreckt. Durch einen solche zusätzlichen rotatorischen Freiheitsgrad zwischen Positioniersteg und Druckstrebe kann der Tragekomfort weiter erhöht werden, insbesondere wenn der genannte Positioniersteg am Ende der Druckstrebe angeordnet und der Großzehe zugeordnet ist.

Die Druckstrebe kann grundsätzlich oberhalb der Zehen angeordnet sein und sich über diese hinweg erstrecken, so dass die Zehen auf der Fußunterseite unbehindert am Boden abrollen können bzw. auf das Fußbett eines Schuhs treten können.

In alternativer Weiterbildung der Erfindung kann jedoch vorgesehen sein, dass sich die Querstrebe auf der Unterseite der Zehen über diese hinweg bis zu der zu korrigierenden Zehe erstreckt, wodurch ein Scheuern an den Zehengelenkskuppen auf der Zehenoberseite gänzlich vermieden ist. Um dennoch auch auf der Unterseite ein Scheuern zu vermeiden, kann in Weiterbildung der Erfindung vorgesehen sein, dass die Querstrebe im Wesentlichen stabförmig ausgebildet ist und sich im Bereich der Zehengrundrille erstreckt, die zwischen den Zehen und dem Fußballen vorgesehen ist. Durch Ausnutzen der Konkavität im Bereich der Zehengrundrille bleiben sowohl der Fußballen als auch die den Bodenkontakt bewirkenden Zehenkuppen frei, wodurch ein dem Barfußlaufen ähnliches Laufgefühl erhalten bleibt.

Alternativ zu einer solchen stabförmigen Ausbildung der Querstrebe kann die genannte Querstrebe auch plattenförmig ausgebildet sein und eine Zehentrittplatte bilden. Eine solche Ausbildung der Querstrebe gibt dieser eine besonders hohe Verbindungssteifigkeit, wodurch nicht nur Druckkräfte auf die zu korrigierende Zehe ausgeübt werden können, sondern auch ein Korrekturmoment eingeleitet werden kann. Zudem verteilt sich die Beanspruchung der Querstrebe, wodurch diese aus einem besonders leichten Material und besonders dünn ausgebildet werden kann.

Um einen hohen Tragekomfort zu erzielen, ist dabei vorteilhafterweise in jedem Fall vorgesehen, dass mit Ausnahme der genannten Querstrebe die gesamte Fußunterseite frei bleibt, d.h. die Gelenkschiene erstreckt sich ausschließlich auf der Fußaußenseite im Bereich des Außenrists bzw. der Seitenflanke zwischen Fußoberseite und Fußunterseite. Die Schienenschenkel der Gelenkschiene können dabei insbesondere streifenförmige, plattenartige Stege bilden, die in der angelegten Gebrauchsstellung eine im Wesentlichen aufrechte Ausrichtung einnehmen. Vorteilhafterweise sind dabei die genannten Schienenschenkel einschließlich des Gelenks zwischen ihnen an die Wölbung der Fußaußenseite angepasst und in geeigneter Weise leicht konkav konturiert, um eine flächige Verteilung der Kräfte zu erreichen. Insbesondere kann im Bereich der Gelenkachse auf der Gelenkinnenseite eine konkave schalenförmige Wölbung vorgesehen sein, die auf dem Zehengrundgelenk der kleinen Zehe sitzt. Die Gelenkachse des Gelenks der Gelenkschiene erstreckt sich vorteilhafterweise im Wesentlichen koaxial zu der Grundgelenksachse der kleinen Zehe.

Um eine individuell anpassbare Korrektur einer jeweiligen Zehe vornehmen zu können und ggf. die Korrekturkräfte nach gewisser Zeit graduell nachstellen zu können, ist in Weiterbildung der Erfindung vorgesehen, dass die Querstrebe, die sich von dem vorderen Schienenschenkel aus erstreckt, längenveränderbar ausgebildet ist. Im Falle der zuvor beschriebenen stabförmigen Ausbildung der Querstrebe kann diese beispielsweise einen in eine Schraubhülse einschraubbaren Schraubbolzen umfassen, der einerseits eine zusätzliche Gelenkachse für den Positioniersteg realisiert und andererseits durch beispielsweise Herausschrauben vor dem Anlegen der Zehenbandage verlängert werden kann, um die Korrekturwirkung zu erhöhen.

Alternativ kann bei plattenförmiger Ausbildung der Querstrebe vorgesehen sein, dass zwei plattenförmige Querstrebenstücke yrelativ zueinander in verschiedenen Positionen fixierbar sind. Hierzu können grundsätzlich verschiedene Verstell- und/oder Fixiermittel vorgesehen werden. Nach einer vorteilhaften Ausführung der Erfindung kann zwischen den beiden Querstrebenstücken eine Haken- und Flauschteilverbindung, wie sie unter dem Handelsnamen Klettverschluss bekannt ist, vorgesehen sein. Hierdurch kann eine stufenlose Verstellung der Querstrebe erreicht werden, und zwar sowohl hinsichtlich der Länge der Querstrebe als auch hinsichtlich der exakten Positionierung des zwischen die Zehen vorspringenden Positionierstegs in Fußlängsrichtung als auch hinsichtlich der Winkelausrichtung.

Um eine präzise Fehlstellungskorrektur zu erreichen, ist die Mittelfußbandage und/oder die Zehenbandage längenveränderbar ausgebildet, so dass sie fest und mit dem notwendigen Zug unabhängig von der jeweiligen Ausbildung des Fußes an diesen anlegbar ist. In vorteilhafter Weiterbildung der Erfindung kann hierbei vorgesehen sein, dass die Bandagenlänge sowohl von der Fußunterseite her als auch von der Fußoberseite her veränderbar ist. In Weiterbildung der Erfindung kann der hintere Schienenschenkel der Gelenkschiene zumindest zwei Durchschlauföffnungen besitzen, durch die die Mittelfußbandage mit ihren beiden Bandagenenden durchgeschlauft ist. Die beiden durchgeschlauften Bandagenenden werden vorteilhafterweise zurückgeschlagen und mittels geeigneter Verschlussmittel beispielsweise in Form eines Haken- und Flauschteils an einem noch nicht durchgeschlauften Bandagenabschnitt fixiert, so dass die Bandagenenden unabhängig voneinander gespannt und fixiert werden können. Diese doppelte Spann- und Fixierbarkeit ermöglicht es, die Gelenkschiene präzise in exakt der richtigen Höhe auf der Fußaußenseite zu fixieren und zu spannen.

In entsprechender Weise kann auch die Zehenbandage insbesondere für die Großzehe in entsprechender Weise an dem Positioniersteg zweifach durchgeschlauft und mit entsprechenden Spann- und/oder Fixiermitteln versehen sein, so dass auch die Zehe in entsprechender Weise präzise an der Querstrebe befestigt werden kann.

Die vorliegende Erfindung wird nachfolgend anhand bevorzugter Ausführungsbeispiele und zugehöriger Zeichnungen näher erläutert. In den Zeichnungen zeigen:
- Fig. 1:: eine perspektivische, schematische Ansicht der orthopädischen Vorrich- tung zur Korrektur einer Großzehenfehlstellung nach einer vorteilhaften Ausführung der Erfindung, wonach die von dem vorderen Schienen- schenkel getragene Querstrebe stabförmig ausgebildet ist,
- Fig. 2:: eine schematische, perspektivische Ansicht der orthopädischen Vorrich- tung aus Fig. 1 schräg von vorne, die die Innenseite der Gelenkschiene zeigt,
- Fig. 3:: eine perspektivische Ansicht der an einen Fuß angelegten Vorrichtung aus den vorhergehenden Figuren, die die Fußaußenseite mit der daran befestigten Gelenkschiene zeigt,
- Fig. 4:: eine Draufsicht auf einen Fuß von oben her mit der angelegten orthopä- dischen Vorrichtung aus den vorhergehenden Figuren,
- Fig. 5:: eine Draufsicht auf die orthopädische Vorrichtung aus den vorhergehen- den Figuren von oben her ohne Fuß,
- Fig. 6:: eine Draufsicht auf die Fußunterseite mit der am Fuß befestigten ortho- pädischen Vorrichtung aus den vorhergehenden Figuren,
- Fig. 7:: eine Draufsicht auf einen Fuß von oben her mit einer daran befestigten orthopädischen Vorrichtung nach einer weiteren vorteilhaften Ausführung der Erfindung, wonach die Querstrebe als Zehentrittplatte ausgebildet ist,
- Fig. 8:: eine Frontansicht des Fußes und der daran befestigten orthopädischen Vorrichtung aus Fig. 7, die die längenveränderbare und positionsver- stellbare Ausbildung der plattenförmig ausgebildeten Querstrebe zeigt, und
- Fig. 9:: eine Draufsicht auf eine orthopädische Vorrichtung nach einer weiteren Ausführung der Erfindung, wonach die Querstrebe in Form einer Tritt- platte ausgebildet ist und mehrere Positionierstege zur Korrektur mehre- rer Zehen besitzt.

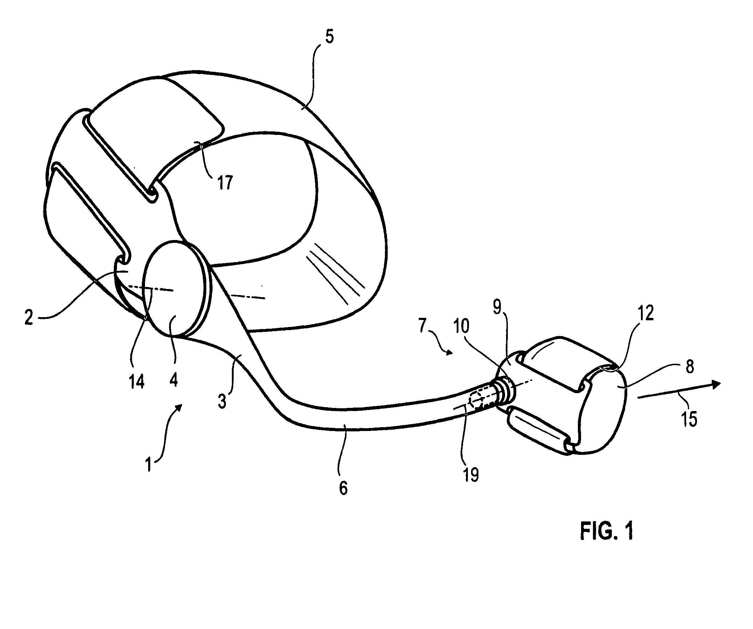

Die in Fig. 1 gezeichnete Zehenkorrekturvorrichtung umfasst eine Gelenkschiene 1 mit zwei Schienenschenkeln 2 und 3, die miteinander durch ein Gelenk 4 verbunden sind. Der hintere Schienenschenkel 2 ist mit einer Mittelfußbandage 5 verbunden, mittels derer der hintere Schienenschenkel 2 am Mittelfuß befestigt werden kann. Der vordere Schienenschenkel 3 trägt an seinem vorderen Ende eine Querstrebe 6, die sich quer zur Längsrichtung der Gelenkschiene 1 zur Fußinnenseite hin erstreckt und an ihrem inneren Ende 7 eine Zehenbandage 8 trägt, mittels derer die Großzehe an der genannten Querstrebe 6 befestigt werden kann.

In der gezeichneten Ausführungsform ist die genannte Querstrebe 6 stabförmig ausgebildet und integral einstückig an den vorderen Schienenschenkel 3 angeformt, der an seinem vorderen Ende eine Abkröpfung umfasst, von der ausgehend sich die genannte Querstrebe 6 unter den Zehen hindurch zur Fußinnenseite hin erstreckt. Die Querstrebe 6 ist dabei derart ausgebildet und angeordnet, dass sie sich im Bereich der Zehengrundrille zwischen den Zehen und dem Fußballen erstreckt und an die konkave Rinne anschmiegt, die sich unter dem Fuß zwischen Zehen und Fußballen befindet.

An ihrem inneren Ende trägt die Querstrebe 6 einen Positioniersteg 9, der sich im Wesentlichen aufrecht in Fußlängsrichtung erstreckt und in den Zwischenraum zwischen Großzehe und benachbarter Zehe hinein vorspringt, so dass dieser Positioniersteg 9 gegen die Außenseite der Großzehe drückt, um hierdurch die Großzehe zur Fußinnenseite hin zu drücken.

Der genannte Positioniersteg 9 kann starr mit der Querstrebe 6 verbunden sein, insbesondere integral daran angeformt sein. Alternativ kann jedoch auch vorgesehen sein, dass der genannte Positioniersteg 9 gelenkig an der Querstrebe 6 gelagert ist, insbesondere schwenkbar um eine Querachse, die sich im Wesentlichen parallel zur Erstreckung der Querstrebe 6 erstreckt. Beispielsweise kann die Querstrebe 6 an ihrem inneren Ende 7 eine Schraubhülse bilden, in die ein Schraubolzen 10 eingeschraubt ist, an dem wiederum starr der genannte Positioniersteg 9 befestigt ist.

An dem genannten Positioniersteg 9 ist die Zehenbandage 8 befestigt. Hierzu kann der Positioniersteg 9 zwei schlitzförmige Durchschlauföffnungen besitzen, durch die die Großzehenbandage 8 mit ihren beiden Enden durchgeschlauft sein kann. Die umgeschlagenen Enden werden mittels Klettverschlüssen 11 auf den Außenseiten der Zehenbandage 8 fixiert.

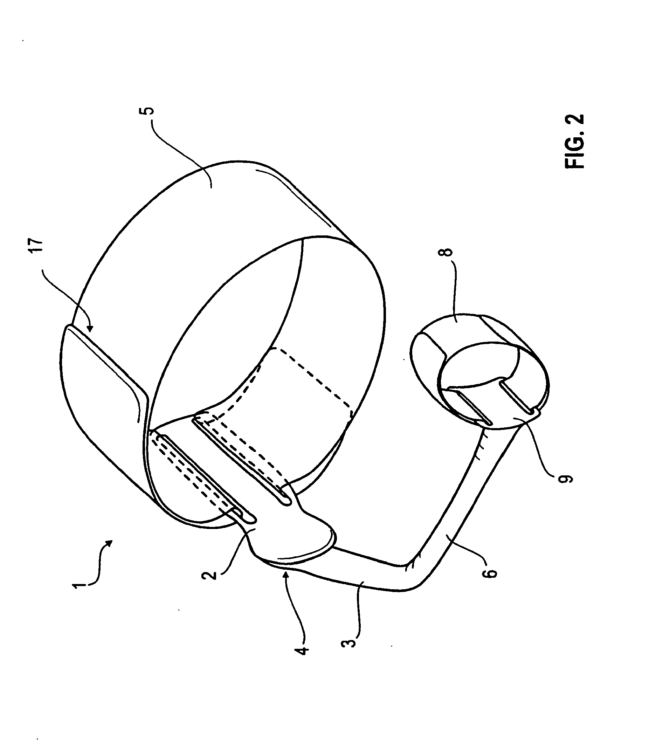

In ähnlicher Weise ist die Mittelfußbandage 5 durch den hinteren Schienenschenkel 2 durchgeschlauft, der hierzu ebenfalls schlitzförmige Durchschlauföffnungen aufweist. Auch die Mittelfußbandage 5 kann vorteilhafterweise mit ihren beiden Enden gespannt und fixiert werden, wozu in entsprechender Weise Klettverschlüsse 12 vorgesehen sein können, vgl. Fig. 2.

Alternativ oder zusätzlich könnten die Mittelfußbandage 5 und/oder die Zehenbandage 8 auch elastisch ausgebildet sein, um eine ausreichende Befestigungswirkung am Mittelfuß bzw. am Zehen zu erreichen.

Wie Figuren 1 und 2 zeigen, sind die beiden Schienenschenkel 2 und 3 - grob gesprochen - im Wesentlichen plattenförmig bzw. stegförmig ausgebildet, so dass sie sich ausschließlich auf der Fußaußenseite erstrecken, wobei sie im am Fuß angelegten Zustand eine im Wesentlichen aufrechte Ausrichtung einnehmen. Die Innenseite der Schienenschenkel 2 und 3 ist dabei leicht rinnenförmig konkav konturiert, um sich an die Außenseite des Fußspanns anzuschmiegen.

Im Bereich des Gelenks 4 überlappen die beiden Schienenschenkel 2 und 3 einander, wobei an die genannten Schienenschenkel 2 und 3 jeweils Gelenksschalen 13 angeformt sind, die pfannenförmig übereinander liegen und die Gelenkachse 14 des Gelenks 4 definieren, die sich im Wesentlichen koaxial zu der Gelenkachse des Grundgelenks der kleinen Zehe erstreckt. Die Innenseite des Gelenks 4 ist durch die Ausbildung der Gelenkschalen 13 leicht pfannenförmig konkav gewölbt, wodurch sich die Gelenkschiene 1 passgenau auf die Wölbung des Kleinzehengrundgelenks auf der Fußaußenseite schmiegt. Hierdurch kann einerseits ein angenehmer Tragekomfort erreicht und andererseits ein Verrutschen der Gelenkschiene verhindert werden.

Im angelegten Zustand erlaubt das Gelenk 4 ein angenehmes Tragen der Vorrichtung auch beim Laufen. Werden beim Abrollen des Fußes die Zehen abgeknickt, kann der vordere Schienenschenkel 3 gegenüber dem hinteren Schienenschenkel 2 entsprechend abknicken. Zudem kann durch die zusätzliche Gelenkachse zwischen dem Positioniersteg 9 und der Querstrebe 6 besonders die Großzehe besonders leicht abgewinkelt werden. Dieser zusätzliche rotatorische Freiheitsgrad des Positionierstegs 9 gleicht einen etwaigen Versatz zwischen der Gelenkachse 14 des Gelenks 4 und dem Gelenk der Großzehe aus.

Wie aus den Figuren 1 und 2 leicht ersichtlich, kann die Korrekturwirkung, d.h. die Größe der auf die Großzehe wirkenden Kraft leicht dadurch eingestellt werden, dass vor dem Anlegen der Vorrichtung der Positioniersteg 9 mittels des Schraubbolzens 10 mehr oder weniger stark aus der Schraubhülse der Querstrebe herausgeschraubt wird, wodurch die Länge der Querstrebe verändert wird.

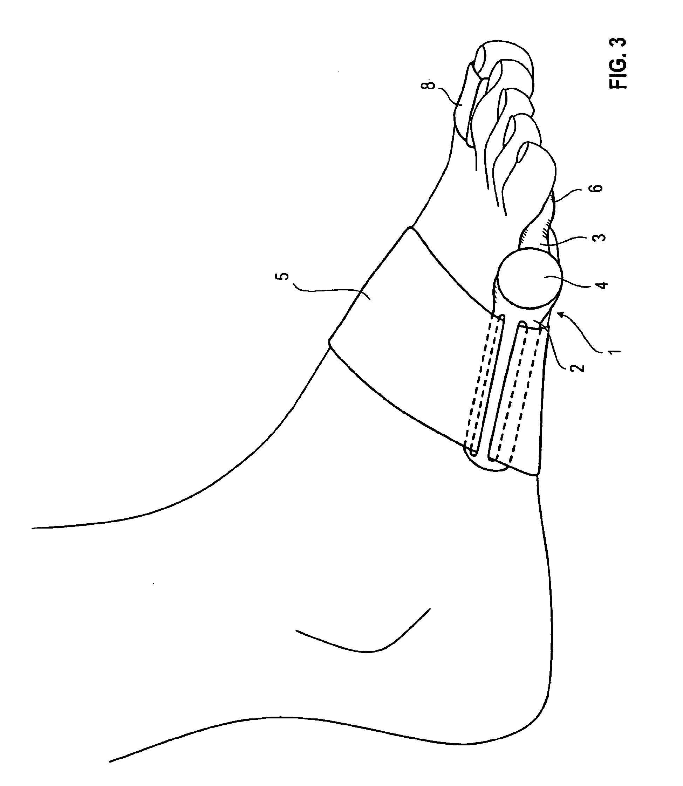

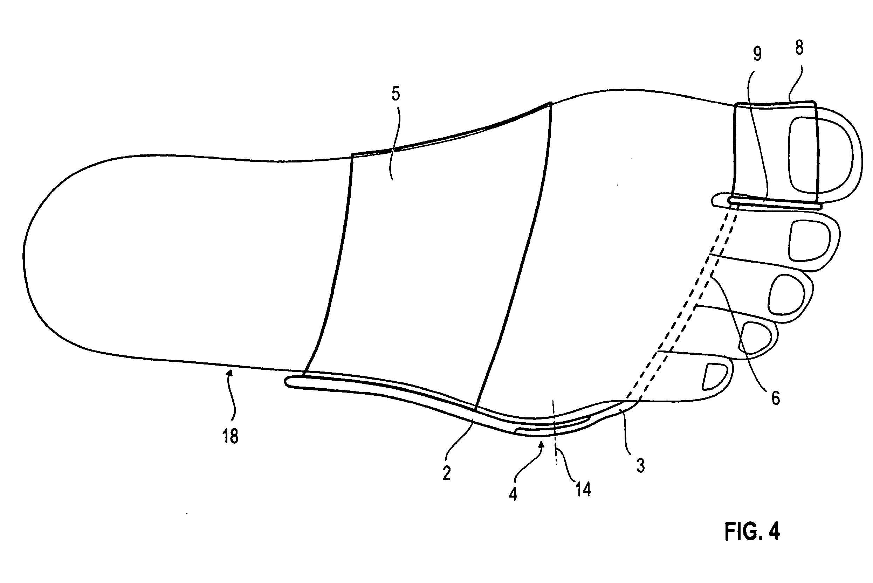

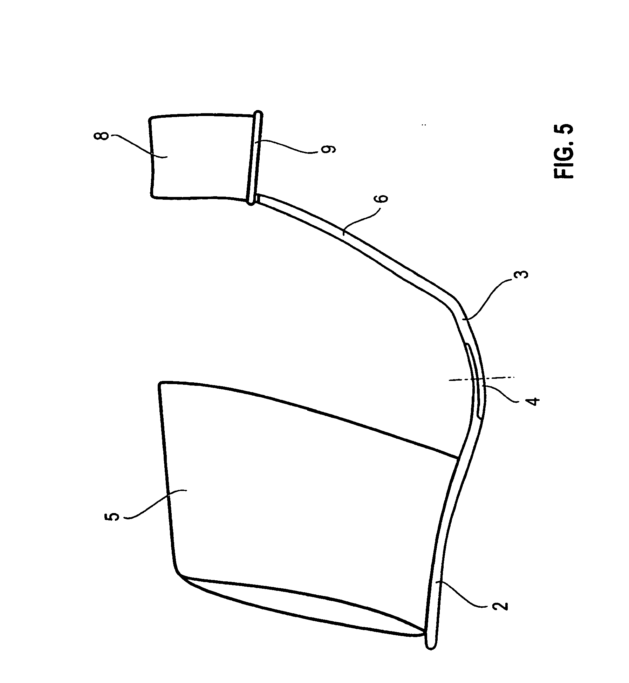

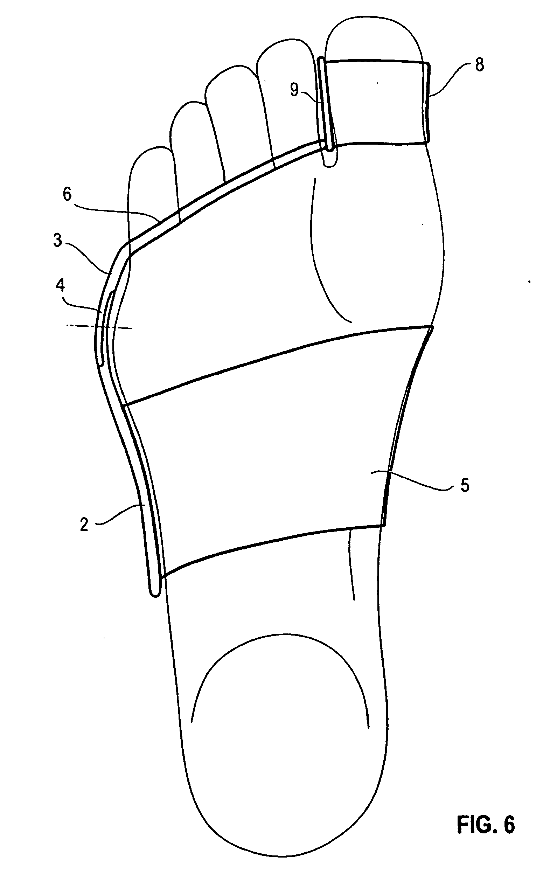

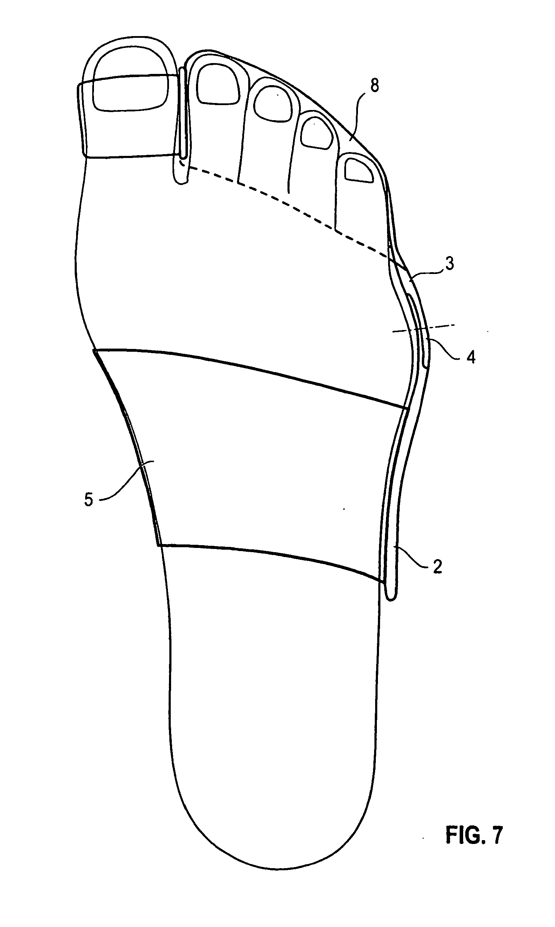

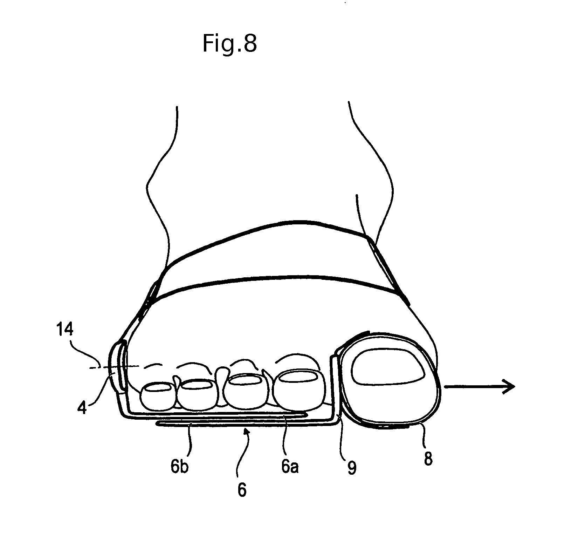

Alternativ zu der in den Figuren 1 bis 6 gezeigten Ausbildung kann die Querstrebe 6 auch plattenförmig ausgebildet sein und eine Trittplatte bilden, auf der die Zehen auftreten. Bei der Ausbildung gemäß den Figuren 7 und 8 ist die genannte Platte, die die Querstrebe 6 bildet, am vorderen Ende des vorderen Schienenschenkels 3 befestigt. An ihrem inneren Ende 7 umfasst die Trittplatte eine Abfalzung nach oben, die sich als Positioniersteg 9 zwischen der Großzehe und der benachbarten Zehe in den dort vorgesehen Zwischenraum hinein erstreckt, um die Großzehe zur Fußinnenseite hin zu drücken, wie dies der Pfeil 15 in Fig. 8 verdeutlicht. Der genannte Positioniersteg 9 ist dabei in der zuvor beschriebenen Weise mittels der Zehenbandage 8 an der Großzehe fixiert.

Wie Fig. 8 zeigt, ist die Trittplatte der Querstrebe 6 längen- und positionseinstellbar ausgebildet. Die genannte Trittplatte umfasst hierzu zwei Plattenstücke 6a und 6b, die aufeinander sitzen und in verschiedenen Positionen relativ zueinander fixiert werden können. Hierzu sind Positionseinstell- und Fixiermittel 16 vorgesehen, die in der gezeichneten Ausführung aus einem Haken- und Flauschteilverschluss 17, insbesondere Klettverschluss, bestehen. Hierdurch können die beiden Plattenteile stufenlos zueinander verstellt und in beliebigen Positionen relativ zueinander fixiert werden, wodurch die Stellung des die Korrektur bewirkenden Positionierstegs 9 fein einstellbar ist.

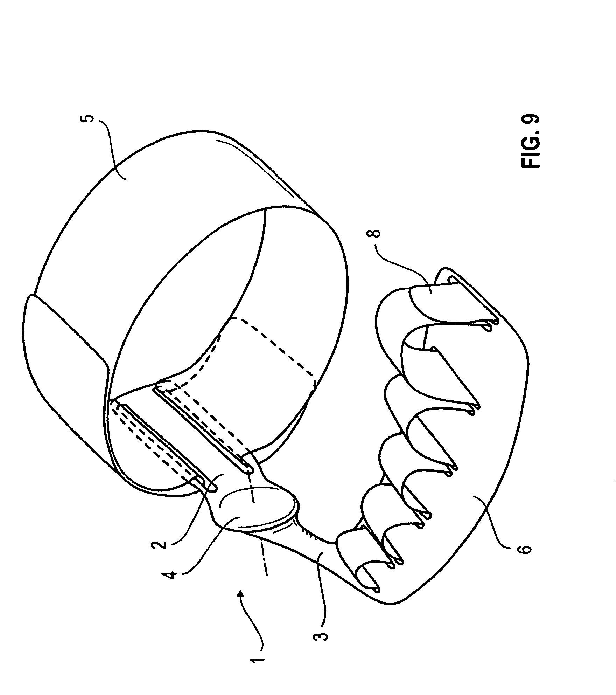

Bei der in Fig. 9 gezeichneten Ausführung umfasst die Querstrebe 6 mehrere Positionierstege 9, die zwischen mehreren Zehenpaaren hinein vorspringen, um mehrere Zehen gleichzeitig hinsichtlich ihrer Stellung zu korrigieren. Im Übrigen entspricht die Ausführung der Ausführung nach Fig. 8.

## Patentansprüche

1. Orthopädische Vorrichtung zur Korrektur von Zehenfehlstellungen, mit einer Gelenkschiene (1), die zwei um eine liegende Gelenkachse (14) miteinander gelenkig verbundene Schienenschenkel (2, 3) aufweist, von denen, in Gebrauch, ein hinterer Schienenschenkel (2) mittels einer Mittelfußbandage (5) am Mittelfuß befestigbar und von denen ein vorderer Schienenschenkel (3) mittels einer Zehenbefestigung an einer Zehe befestigbar ist, **dadurch gekennzeichnet, dass** sich die beiden Schienenschenkel (2, 3) auf der Fußaußenseite (18) erstrecken und der vordere Schienenschenkel (3) eine Querstrebe (6) trägt, die sich quer zu den Schienenschenkeln (2 und 3) über und/oder unter zumindest der kleinen Zehe hinweg zur Fußinnenseite hin zu einem zu korrigierenden Zehen erstreckt, die mittels der genannten Zehenbefestigung an der Querstrebe (6) befestigbar ist.

2. Orthopädische Vorrichtung nach dem vorhergehenden Anspruch, wobei die genannte Querstrebe (6) als Druckstrebe ausgebildet ist und Mittel zur Beaufschlagung der zu korrigierenden Zehe mit einer zur Fußinnenseite hin gerichteten Kraft besitzt, wobei die Druckstrebe zumindest einen zwischen zwei benachbarten Zehen hinein vorspringenden Positioniersteg (9) zur lateralen Zehenpositionierung aufweist.

3. Orthopädische Vorrichtung nach dem vorhergehenden Anspruch, wobei die Druckstrebe mehrere voneinander beabstandete Positionierstege (9) trägt, die zwischen verschiedenen Zehenpaaren hinein vorspringen.

4. Orthopädische Vorrichtung nach einem der beiden vorhergehenden Ansprüche, wobei der zumindest eine Positioniersteg (9) um eine liegende, sich quer zu den Schienenschenkeln (2, 3) erstreckenden Drehachse (19) an der Querstrebe (6) drehbar gelagert ist.

5. Orthopädische Vorrichtung nach einem der vorhergehenden Ansprüche, wobei die Gelenkachse (14) zwischen den beiden Schienenschenkeln (2, 3) sich im Wesentlichen koaxial zu einer Kleinzehengrundgelenksachse erstreckt.

6. Orthopädische Vorrichtung nach einem der vorhergehenden Ansprüche, wobei sich die Querstrebe (6) auf einer Zehenunterseite erstreckt.

7. Orthopädische Vorrichtung nach einem der vorhergehenden Ansprüche, wobei die Querstrebe (6) stabförmig ausgebildet ist und sich in der Zehengrundrille zwischen Zehen und Fußballen unter den Zehen erstreckt.

8. Orthopädische Vorrichtung nach Anspruch 7, wobei die Querstrebe (6) plattenförmig ausgebildet ist und eine Zehentrittplatte bildet.

9. Orthopädische Vorrichtung nach einem der vorhergehenden Ansprüche, wobei die Querstrebe (6) eine Großzehenpositionierfläche aufweist, die mittels einer Großzehenbandage am Großzeh fixierbar ist.

10. Orthopädische Vorrichtung nach einem der vorhergehenden Ansprüche, wobei die Querstrebe (6) längenveränderbar ausgebildet ist und Längeneinstellmittel zum Fixieren der Querstrebe (6) in einer jeweiligen Länge vorgesehen sind.

11. Orthopädische Vorrichtung nach einem der vorhergehenden Ansprüche, wobei die Querstrebe (6) Positionseinstellmittel zur Einstellung der Position einer Zehenanlagefläche, mit der die Querstrebe (6) an der zu korrigierenden Zehe anliegt, gegenüber dem vorderen Schienenschenkel (3) aufweist.

12. Orthopädische Vorrichtung nach dem vorhergehenden Anspruch, wobei die Querstrebe (6) eine Schraubhülse aufweist, in die ein Schraubbolzen einschraubbar ist, der die genannte Zehenanlagefläche trägt.

13. Orthopädische Vorrichtung nach einem der vorhergehenden Ansprüche, wobei die Querstrebe (6) zwei zueinander verstellbare Trittplattenteile aufweist, die mittels einer Fixiereinrichtung, insbesondere einer Haken- und Flauschteilverbindung, relativ zueinander fixierbar sind.

14. Orthopädische Vorrichtung nach einem der vorhergehenden Ansprüche, wobei eine Fußsohle von der Gelenkschiene (1) ausgespart und/oder freigelassen ist und/oder sich die Schienenschenkel (2, 3) mit Ausnahme der Querstrebe (6) ausschließlich auf der Außenseite des Fußes erstrecken.

15. Orthopädische Vorrichtung nach einem der vorhergehenden Ansprüche, wobei die Mittelfußbandage (5) längeneinstellbar ausgebildet ist, wobei der hintere Schienenschenkel (2) zumindest zwei Durchschlauföffnungen besitzt, durch die die Mittelfußbandage (5) mit ihren beiden Bandagenenden her durchgeschlauft ist, wobei die durchgeschlauften Bandagenenden mittels Verschlussmitteln, vorzugsweise einem Haken- und Flauschteilverschluss, unabhängig voneinander spann- und/oder fixierbar sind.

## Claims

1. An orthopaedic apparatus for correcting defective positions of toes, having a joint splint (1) which has two splint parts (2, 3) which are pivotally connected to one another about a horizontal joint axis (14) and of which, in use, a rear splint part (2) can be fastened to the metatarsus by means of a metatarsal bandage (5), and of which a front splint part (3) can be fastened to a toe by means of a toe fastener, **characterised in that** the two splint parts (2, 3) extend on the lateral side of the foot (18) and the front splint part (3) bears a crossmember (6) which extends transversely to the splint parts (2 and 3) above and/or below at least the small toe toward the medial side of the foot up to a toe to be corrected which can be fastened to the crossmember (6) by means of the named toe fastener.

2. An orthopaedic apparatus in accordance with the preceding claim, wherein the named crossmember (6) is made as a strut and has means for application of a force directed to the medial side of the foot on the toe to be corrected, wherein the strut has at least one positioning web (9) which projects between two adjacent toes for the lateral positioning of the toes.

3. An orthopaedic apparatus in accordance with the preceding claim, wherein the strut bears a plurality of mutually spaced apart positioning webs (9) which project between different pairs of toes.

4. An orthopaedic apparatus in accordance with one of the two preceding claims, wherein the at least one positioning web (9) is rotatably supported about a horizontal axis of rotation (19) at the crossmember (6) which extends transversely to the splint parts (2, 3).

5. An orthopaedic apparatus in accordance with one of the preceding claims, wherein the joint axis (14) extends between the two splint parts (2, 3) substantially coaxially to a base joint axis of the small toe.

6. An orthopaedic apparatus in accordance with one of the preceding claims, wherein the crossmember (6) extends on a lower side of the toe.

7. An orthopaedic apparatus in accordance with one of the preceding claims, wherein the crossmember (6) is made in bar shape and extends beneath the toes in the groove of the base of the toe between the toes and the ball of the foot.

8. An orthopaedic apparatus in accordance with claim 7, wherein the crossmember (6) is formed in plate shape and forms a foot plate for a toe.

9. An orthopaedic apparatus in accordance with one of the preceding claims, wherein the crossmember (6) has a positioning surface for the big toe which can be fixed to the big toe by means of a big toe bandage.

10. An orthopaedic apparatus in accordance with one of the preceding claims, wherein the crossmember (6) is made longitudinally changeable and length setting means are provided for fixing the crossmember (6) in a respective length.

11. An orthopaedic apparatus in accordance with one of the preceding claims, wherein the crossmember (6) has position setting means for setting the position of a toe contact surface with which the crossmember (6) contacts the toe to be corrected opposite the front splint part (3).

12. An orthopaedic apparatus in accordance with the preceding claim, wherein the crossmember (6) has a screw sleeve into which a screw bolt can be screwed which supports the named toe contact surface.

13. An orthopaedic apparatus in accordance with one of the preceding claims, wherein the crossmember (6) has two mutually adjustable foot plate parts which can be fixed relative to one another by means of a fixing device, in particular a hook and loop part connection.

14. An orthopaedic apparatus in accordance with one of the preceding claims, wherein a sole of the foot is left out and/or left free by the joint splint (1) and/or the splint parts (2, 3) extend, except for the crossmember (6), only on the lateral side of the foot.

15. An orthopaedic apparatus in accordance with one of the preceding claims, wherein the metatarsal bandage (5) is made longitudinally adjustable, wherein the rear splint part (2) has at least two loop openings through which the two bandage ends of the metatarsal bandage (5) are looped, wherein the looped through bandage ends can be tightened and/or fixed independently of one another by means of closure means, preferably a hook and loop closure.

## Revendications

1. Dispositif orthopédique destiné à corriger les malpositions d'orteils, avec une attelle articulaire (1), qui présente deux montants d'attelle reliés l'un à l'autre de manière articulée (2, 3) autour d'un axe articulé couché (14), parmi lesquels, pendant l'utilisation, un montant d'attelle arrière (2) est fixable au métatarse à l'aide d'un bandage de métatarse (5) et parmi lesquels un montant d'attelle avant (3) est fixable au moyen d'une fixation d'orteil à un orteil, **caractérisé en ce que** les deux montants d'attelle (2, 3) s'étendent sur le côté externe du pied (18) et **en ce que** le montant d'attelle avant (3) porte une entretoise (6), qui s'étend transversalement par rapport aux montants d'attelle (2 et 3) par le dessus et/ou par le dessous au-moins du petit orteil vers le côté interne du pied jusqu'à un orteil à corriger, qui est fixable au moyen de ladite fixation d'orteil à l'entretoise (6).

2. Dispositif orthopédique selon la revendication précédente, ladite entretoise (6) étant constituée comme arc-boutant et dotée de moyens d'alimentation de l'orteil à corriger par une force dirigée vers le côté interne du pied, l'arc-boutant présentant au moins une barre de positionnement (9) ressautant au-dedans entre deux orteils voisins vers le positionnement latéral d'orteil.

3. Dispositif orthopédique selon la revendication précédente, l'arc-boutant portant plusieurs barres de positionnement (9) écartées les unes des autres, qui ressautent au-dedans entre différentes paires d'orteils.

4. Dispositif orthopédique selon une quelconque des deux revendications précédentes, au moins l'une des barres de positionnement (9) étant sur paliers pivotants sur l'entretoise (6) autour d'un axe pivotant (19) couché s'étendant transversalement par rapport aux montants d'attelle (2, 3).

5. Dispositif orthopédique selon une quelconque des revendications précédentes, l'axe articulé (14) s'étendant pour l'essentiel de manière coaxiale entre les deux montants d'attelle (2, 3) vers un axe articulé de base du petit orteil.

6. Dispositif orthopédique selon une quelconque des revendications précédentes, l'entretoise (6) s'étendant vers un côté inférieur de l'orteil.

7. Dispositif orthopédique selon une quelconque des revendications précédentes, l'entretoise (6) étant façonnée en forme de barre et s'étendant au-dessous des orteils dans l'interstice de base des orteils entre les orteils et les éminences du gros et du petit orteil.

8. Dispositif orthopédique selon la revendication 7, l'entretoise (6) étant façonnée en forme de plaque et constituant un marchepied d'orteil.

9. Dispositif orthopédique selon une quelconque des revendications précédentes, l'entretoise (6) présentant une surface de positionnement du gros orteil, qui est fixable au gros orteil à l'aide d'un bandage de gros orteil.

10. Dispositif orthopédique selon une quelconque des revendications précédentes, l'entretoise (6) étant constituée de manière modifiable en longueur et des moyens de réglage de la longueur pour la fixation de l'entretoise (6) étant prévus à une longueur respective.

11. Dispositif orthopédique selon une quelconque des revendications précédentes, l'entretoise (6) présentant des moyens de réglage de la position destinés au réglage de la position d'une surface d'appui des orteils, avec laquelle l'entretoise (6) s'appuie sur les orteils à corriger par rapport au montant d'attelle avant (3).

12. Dispositif orthopédique selon la revendication précédente, l'entretoise (6) présentant un manchon fileté, dans lequel un boulon fileté est vissable, qui porte ladite surface d'appui des orteils.

13. Dispositif orthopédique selon une quelconque des revendications précédentes, l'entretoise (6) présentant deux éléments de marchepied réglables l'un par rapport à l'autre, qui sont fixables l'un en relation avec l'autre à l'aide d'un dispositif de fixation, notamment d'un lien à crochets et à éléments en velours.

14. Dispositif orthopédique selon une quelconque des revendications précédentes, une plante étant évidée et/ou laissée libre à partir de l'attelle articulaire (1) et/ou les montants d'attelle (2, 3) s'étendant, à l'exception de l'entretoise (6), exclusivement sur le côté externe du pied.

15. Dispositif orthopédique selon une quelconque des revendications précédentes, le bandage de métatarse (5) étant constitué de manière réglable en longueur, le montant d'attelle arrière (2) possédant au moins deux ouvertures de passants, à travers lesquelles le bandage de métatarse (5) est suspendu par ses deux extrémités de bandage, les extrémités de bandages suspendues étant serrables et/ou fixables indépendamment l'une de l'autre à l'aide d'éléments d'immobilisation, de préférence une immobilisation à crochets et à éléments en velours.
